# EUROPÄISCHE PATENTANMELDUNG

(11) **EP 4 840 286 A1**
(43) Veröffentlichungstag der Anmeldung: **18.11.2026**
(21) Anmeldenummer: 26166695.2
(22) Anmeldetag: 23.03.2026
(51) Int. Cl.: A01D 41/127, G01B 11/04, G01N 21/85, G01N 21/359, G01N 21/3563

(54) **VERFAHREN ZUR ONLINE-ANALYSE VON ERNTEGUT**

(30) Priorität: 14.05.2025 DE 102025118541
(71) Anmelder: CLAAS Selbstfahrende Erntemaschinen GmbH, 33428 Harsewinkel (DE)
(72) Erfinder: Marbach, Ralf, 33775 Versmold (DE)
(74) Vertreter: CLAAS Gruppe

(57) **Zusammenfassung**

Die vorliegende Erfindung betrifft ein Verfahren zur Online-Analyse von Erntegut (2), welches von einem Mähdrescher (1) verarbeitet wird, wobei ein Erntegutstrom (9) von einem Kornelevator (10) zu einem Korntank (5) gefördert wird, wobei mit einer am Kornelevator (10) in Bypass-Anordnung angeordneten Probenentnahmevorrichtung (12) ein Probenkornstrom (13) entnommen und Ernteguteigenschaften der enthaltenen Körner (8, 8a) von einer Messanordnung (20) der Probenentnahmevorrichtung (12) mit einer Auswerteeinheit (21) ermittelt werden. Dabei erfolgt eine Separation einzelner Körner (8, 8a) als Probenkornstrom (23) aus dem Erntegutstrom (9); Erfassung von Korngrößen der Körner (8, 8a) im Probenkornstrom (13) durch eine optische Sensoranordnung (23) und Einteilung in mehrere Gewichtsklassen; und Bestimmung von Inhaltsstoffen des Probenkornstromes (13) durch einen optischen Sensor (27), wobei die generierten Probenkornstromdaten an die Auswerteeinheit (21) übertragen werden, um daraus Informationen zur Proteinverschiebung (PSI) zu bestimmen, welche durch die Auswerteeinheit (21) zur Generierung zumindest einer Handlungsempfehlung für einen nachfolgenden Verarbeitungsprozess des Erntegutes (2) ausgewertet werden.

## Beschreibung

Die vorliegende Erfindung betrifft ein Verfahren zur Online-Analyse von Erntegut gemäß dem Oberbegriff des Anspruches 1. Weiterhin ist ein selbstfahrender Mähdrescher Gegenstand der Erfindung.

Aus der EP 4 179 867 A1 ist ein Verfahren zur Analyse von Erntegut bekannt, welches von einem selbstfahrenden Mähdrescher aufgenommen wird. Zur Analyse von Inhaltsstoffen wird mittels einer Bypass-Vorrichtung ein Erntegut-Teilstrom einem vom Kornelevator geförderten, aus Körnern bestehenden Erntegutstrom entnommen und mittels einer in der Bypass-Vorrichtung angeordneten Messvorrichtung analysiert. Hierzu verwendet die Messvorrichtung einen Nahinfrarotsensor, welcher massenproportionale Absorptionsspektren erfasst. Anhand dieser Messdaten wird die Wassermasse, die Proteinmasse, die Kohlenhydratmasse und/oder die Ölmasse des Erntegut-Teilstromes ermittelt und auf die Masse im Korntank hochgerechnet.

Ausgehend vom vorstehend genannten Stand der Technik liegt der Erfindung die Aufgabe zugrunde, ein Verfahren zur Online-Analyse von Erntegut der eingangs genannten Art weiterzubilden, welches sich durch eine detaillierte Analyse des aufgenommenen Erntegutes während des laufenden Erntevorganges auszeichnet.

Die vorstehend genannte Aufgabe wird durch ein Verfahren zur Online-Analyse von Erntegut mit den Merkmalen des Anspruches 1 gelöst. Weiterhin wird die Aufgabe durch einen selbstfahrenden Mähdrescher gemäß dem Anspruch 12 gelöst. Vorteilhafte Weiterbildungen sind Gegenstand der abhängigen Ansprüche.

Gemäß dem Anspruch 1 wird ein Verfahren zur Online-Analyse von Erntegut vorgeschlagen, welches von einem selbstfahrenden Mähdrescher aufgenommen und behandelt wird, wobei ein Erntegutstrom von einem Kornelevator von einer Förder- und Reinigungsvorrichtung zu einem Korntank des Mähdreschers gefördert wird, wobei mit einer am Kornelevator in Bypass-Anordnung angeordneten Probenentnahmevorrichtung ein Probenkornstrom aus dem durch den Kornelevator geführten Erntegutstrom entnommen und Ernteguteigenschaften der im Probenkornstrom enthaltenen Körner von einer Messanordnung der Probenentnahmevorrichtung mit einer Auswerteeinheit ermittelt werden. Das erfindungsgemäße Verfahren ist gekennzeichnet durch die Verfahrensschritte:
- Separierung einzelner Körner als Probenkornstrom aus dem Erntegutstrom mittels einer Entnahmevorrichtung,
- Erfassung von Korngrößen der Körner im Probenkornstrom durch eine der Entnahmevorrichtung nachgeordnete optische Sensoranordnung und Einteilung in mehrere Gewichtsklassen, und
- Bestimmung von Inhaltsstoffen des vorbeiströmenden Probenkornstromes durch einen der Abscheidevorrichtung nachgeordneten optischen Sensor, wobei die vom optischen Sensor generierten, den erfassten Inhaltsstoffen entsprechenden Probenkornstromdaten an die Auswerteeinheit übertragen werden, um daraus Informationen zur Proteinverschiebung (PSI) zu bestimmen, welche durch die Auswerteeinheit zur Generierung zumindest einer Handlungsempfehlung für einen nachfolgenden Verarbeitungsprozess des Erntegutes ausgewertet werden.

Dabei gibt die Information zur Proteinverschiebung einen erwartbaren Anteil an Protein in der Trockenmasse des vom Mähdrescher bearbeiteten Korns in Relation zur Korngröße wieder. Insbesondere können durch die Bestimmung der jeweiligen Information zur Proteinverschiebung für verschiedene Beladungen des Korntanks diese massengewichtet aufaddiert werden, so dass die Information zur Proteinverschiebung der Gesamttonnage genauer und vollständiger bestimmbar ist.

Bevorzugt kann als zumindest eine Handlungsempfehlung für den nachfolgenden Verarbeitungsprozess ein Vorschlag für eine Fraktionierung des Erntegutes generiert werden. In Abhängigkeit von einer Präferenz kann während des Erntevorgangs durch den Mähdrescher eine messtechnische Fraktionierung zur Einhaltung von Anforderungen an den Proteingehalt in einem späteren Verarbeitungsprozess des Korns, beispielsweise bei der Mehlherstellung oder der Verwendung im Brauprozess, durchgeführt werden. Mit anderen Worten kann der Effekt einer sogenannten scharfen Siebung vorbestimmt werden, bei welcher in einem mechanischen Siebvorgang Körner einer bestimmten Gewichtsklasse aufgrund ihres in Relation zum Gewicht hohen oder niedrigen Proteingehaltes abgeschieden werden, bevor das Erntegut vom Betreiber des selbstfahrenden Mähdreschers oder dem Eigentümer des geernteten Korns weiterveräußert wird.

Insbesondere kann die Fraktionierung in Abhängigkeit von den bestimmten Inhaltsstoffanteilen einer jeweiligen Gewichtsklasse durchgeführt werden.

Gemäß einer Weiterbildung kann die zumindest eine generierte Handlungsempfehlung an eine entfernte Datenbank übertragen werden, in welcher die zumindest eine Handlungsanweisung für autorisierte Dritte abrufbar hinterlegt ist. Der Betreiber des selbstfahrenden Mähdreschers oder der Eigentümer des geernteten Korns können durch eine Registrierung den Zugriff auf die mittels des Verfahrens bestimmten Daten erhalten, um die zumindest eine Handlungsempfehlung abzurufen. Die zumindest eine Handlungsempfehlung ist ein Indikator für den ökonomischen Nutzen eines nachfolgend durchzuführenden Siebvorgangs, um den Proteingehalt durch die gezielte Abscheidung einer Fraktion einer Gewichtsklasse zu verändern, d.h. zu erhöhen oder zu verringern, bevor das Erntegut weiterveräußert wird.

Um die Information zur Proteinverschiebung hinreichend genau bestimmen zu können, werden die von der optischen Sensoranordnung erfassten Körner auf zumindest sechs Gewichtsklassen verteilt. Die Anzahl von zumindest sechs Gewichtsklassen dient der Annäherung der erwartbaren Verteilung der Korngrößen respektive der Verteilung des daraus abgeleiteten Korngewichts bezogen auf den vom Kornelevator geförderten Massedurchsatz.

Insbesondere können einzelne Körner, welche einen erntegutabhängig vorgebbaren Schwellwert für eine Korngröße und/oder einen erntegutabhängig vorgebbaren Schwellwert für ein Korngewicht überschreiten oder unterschreiten, identifiziert und einer gemeinsamen Gewichtsklasse zugeordnet werden. Dadurch kann für Fruchtarten, die sehr kleine Körner aufweisen, wie sie beispielsweise für Raps, Leinsamen oder Gräser, typisch sind, für Fruchtarten, die sich durch Körner kleiner oder mittlerer Größe auszeichnen, wie beispielswese bei Weizen oder Gerste, und für Frucht-arten, für die große Körner typisch sind, beispielsweise Mais oder Soja, eine Maximalgröße oder eine Minimalgröße bzw. ein Maximalgewicht oder ein Minimalgewicht vorgegeben werden, die der Analyse zugrunde gelegt werden. Dies ermöglich ein optisches Sieben des Probenkornstromes in Abhängigkeit von einer Präferenz hinsichtlich der Inhaltsstoffe, um diese an Anforderungen in einem späteren Weiterverarbeitungsprozess des Erntegutes durch vorbereitende Maßnahmen anpassen zu können. Die Bestimmung des Ergebnisses der Fraktionierung kann somit in den Analysevorgang integriert werden.

Weiterhin kann innerhalb eines vorgebaren Messzeitraumes eine vorbestimmte Anzahl an aufeinanderfolgenden Messungen mit einem vorgebbaren Messintervall zur Bestimmung der Korngröße durchgeführt werden, wobei mittels eines Softwarealgorithmus ein Histogramm der Korngrößenverteilung im Probenkornstrom durch die Auswerteeinheit erstellt wird.

Insbesondere kann zur Bestimmung der Information zur Proteinverschiebung eine lineare Regressionsgleichung gemäß der Formel $\left(N{\left(t\right)}_{\left(mx6\right)}\times {\overline{m}}_{\left(6x6\right)}\right) \times {P%}_{\left(6x1\right)} = mP {\left(t\right)}_{\left(mx1\right)}$ erstellt wird, wobei die Matrix N(t) die Anzahl der Körner jeder Messung innerhalb des Messzeitraumes umfasst, die auf eine der zumindest sechs Gewichtsklassen verteilt werden, die Diagonalmatrix *m̅* vorgegebene Werte enthält, die das durchschnittliche Korngewicht jeder der zumindest sechs Gewichtsklasse wiedergeben, der Vektor *mP*(*t*) Werte für die Proteinmasse enthält, die vom Sensor zur Inhaltsstoffanalyse innerhalb eines jeden vorgegebenen Messintervalls für alle im Probenkornstrom enthaltenen Körner gemeinsam gemessen wurden, und der Vektor P% die zu bestimmende Konzentration an Proteingehalt innerhalb jeweils einer der zumindest sechs Gewichtsklassen umfasst, welche für den Vektor P% durch die Auswerteeinheit mittels linearer Regressionsanalyse gelöst wird.

Gemäß einer Weiterbildung können einzelne Körner aus dem Probenkornstrom mittels einer der optischen Sensoranordnung nachgeordneten Abscheidevorrichtung in Abhängigkeit von der durch die Auswerteeinheit durchgeführten Auswertung von Signalen, die von der optischen Sensoranordnung zur ausgeführten Größenunterscheidung bereitgestellt werden, abgeschieden werden. Hierbei kann der Bestimmung der Information über die Proteinverschiebung durch die Auswerteeinheit mittels linearer Regressionsanalyse die gezielte Abscheidung von Körnern einer Gewichtsklasse vorgeschaltet werden. Die anschließende Bestimmung der Information zur Proteinverschiebung durch die Auswerteeinheit beschränkt sich somit auf die Verarbeitung von Daten von zumindest fünf Gewichtsklassen.

Des Weiteren können die Körner in Abhängigkeit vom erntegutabhängig vorgebbaren Schwellwert durch die Abscheidevorrichtung aus dem Probenkornstrom abgeschieden werden.

Bevorzugt können mittels einer dem optischen Sensor vor- oder nachgeschalteten Sensoreinheit, welche einen Sensor oder mehrere Sensoren unterschiedlicher Sensortechnologien umfasst, Parametern bestimmt werden, durch welche auf das Vorhandensein von Bruchkorn, die Korngesundheit, Besatz, Oberflächenbeschaffenheit des Korns und/oder Farbe des Korns im Probenkornstrom geschlossen wird.

Die eingangs gestellte Aufgabe wird des Weiteren durch einen selbstfahrenden Mähdrescher mit den Merkmalen des nebengeordneten Anspruches 12 gelöst.

Gemäß dem Anspruch 12 wird ein selbstfahrender Mähdrescher zur Aufnahme und Behandlung von Erntegut vorgeschlagen, der zur Ausführung des Verfahrens nach einem der vorangehenden Ansprüche 1 bis 11 ausgeführt und eingerichtet ist,
- mit einem Kornelevator zur Förderung eines Erntegutstroms von einer Förder- und Reinigungsvorrichtung zu einem Korntank,
- mit einer am Kornelevator angeordneten Probenentnahmevorrichtung, die zur Entnahme und Bestimmung von Ernteguteigenschaften eines aus dem durch den Kornelevator geführten Erntegutstroms entnommenen Probenkornstromes eingerichtet ist, wobei die in Bypass-Anordnung am Kornelevator angeordnete Probenentnahmevorrichtung eine Messanordnung mit einer Auswerteeinheit aufweist, wobei die Probenentnahmevorrichtung eine rotierend angetriebene Entnahmevorrichtung zur kontinuierlichen Separierung von einzelnen Körnern als Probenkornstrom aus dem Erntegutstrom, eine der Entnahmevorrichtung nachgeordnete optische Sensoranordnung zur Größenerfassung der vereinzelten Körner im Probenkornstrom und einen der optischen Sensoranordnung nachgeordneten optischen Sensor umfasst, der zur Bestimmung der Inhaltsstoffe des Probenkornstromes ausgeführt und eingerichtet ist. Auf die erfindungsgemäßen Ausführungen zum Verfahren zur Online-Analyse von Erntegut darf verwiesen werden.

Durch die rotierend angetriebene Entnahmevorrichtung zur kontinuierlichen Separierung von einzelnen Körnern als Probenkornstrom, der einen im Wesentlichen konstanten Massenstrom vorgebbarer Größe aufweist, aus dem Erntegutstrom wird erreicht, dass der Probenkornstrom im Wesentlichen als eine Einzelschicht aus vereinzelten Körnern ausgebildet ist oder nachfolgend auf einfache Weise als Einzelschicht ausbildbar ist, so dass im Idealfall eine optische Analyse eines jeden vereinzelten Korns im Probenkornstrom durch die Messanordnung ermöglicht wird.

Vereinzelt bedeutet in diesem Kontext, dass die optische Sensoranordnung zur Größenerfassung der vereinzelten Körner im Idealfall jedes der Körner im Probenkornstrom optisch erfassen kann.

Gemäß einer Weiterbildung kann die Entnahmevorrichtung als zumindest ein rotierend angetriebenes Nockenrad mit zumindest einer umlaufenden Nockenreihe oder als ein Zellenrad ausgeführt sein. Mittels des zumindest einen Nockenrades kann, in Abhängigkeit von der Anzahl der Nockenreihen, ein definierter zeitlicher Abstand zwischen der Abgabe der einzelnen Körner eingestellt werden. Das Zellenrad hingegen ermöglicht bei einer bestimmten Drehzahl und Arbeitsbreite einen höheren Durchfluss, d.h. eine höhere Fördermenge einzelner Körner, als das Nockenrad. Das Zellenrad nimmt je Zelle mehr Körner auf als eine Nocke des Nockenrades. Die Verwendung des zumindest einen Nockenrades oder des Zellenrades ermöglicht eine einfache Anpassung durch Austausch an unterschiedliche Fruchtarten, die sich hinsichtlich ihrer durchschnittlichen Korngröße voneinander unterscheiden.

Das zumindest eine Nockenrad oder das Zellenrad wird bevorzugt mit einer Drehzahl angetrieben, um einen Massenstrom im Bereich zwischen 10 g/s und 100 g/s bereitzustellen, mit dem der Probenkornstrom der optischen Sensoranordnung zugeführt wird. Der bevorzugte Massenstrom für den Probenkornstrom liegt zwischen 20 g/s und 50 g/s. Insbesondere bei Massenströmen in diesen Bereichen weisen die beiden wichtigsten Erntegutkulturen, Weizen und Gerste, noch ein Strömungsverhalten auf, welches eine möglichst präzise Bestimmung der Korngrößen der Körner im vorbeiströmenden Probenkornstrom durch die optische Sensoranordnung ermöglicht. Bei Massenströmen im bevorzugten Bereich zwischen 20 g/s und 50 g/s strömen die entnommenen Körner im Wesentlichen in einer eindimensionalen Schicht an der optischen Sensoranordnung vorbei.

Bevorzugt können zwei oder mehr Nockenräder koaxial nebeneinander angeordnet sein, wobei die zumindest eine umlaufende Nockenreihe des jeweiligen Nockenrades einen Versatz in Umfangsrichtung aufweist, oder dass ein Nockenrad mit zwei oder mehr in Umfangsrichtung zueinander versetzt angeordneten Nockenreihen vorgesehen ist. Durch den Versatz der koaxial angeordneten Nockenreihen können die Nockenräder die einzelnen Körner zeitlich versetzt aufnehmen und diese entsprechend an die nachgeordnete optische Sensoranordnung zur Größenerfassung abgeben.

Insbesondere kann die optische Sensoranordnung zumindest eine Lichtschranke, zumindest einen Reflexionssensor und/oder zumindest einen photoelektrischen Sensor umfassen. Die Ausführung der optischen Sensoranordnung als zumindest eine Lichtschranke und/oder als zumindest ein Reflexionssensor kann vorgesehen sein, wenn der Probenkornstrom im Wesentlichen aus einer einzelnen Schicht aus Körnern besteht, die mit im Wesentlichen gleichmäßigem Abstand zueinander die optische Sensoranordnung passieren. Dabei kann die optische Sensoranordnung z.B. als zumindest eine LED-Einweglichtschranke oder als zumindest ein LED- Reflexionssensor ausgeführt sein. In Strömungssituationen, in denen die Schichtdicke des Probenkornstroms ungleichmäßiger und/oder die Abstände zwischen den einzelnen Körnern im Probenkornstrom ungleichmäßig und/oder zu gering sind, ist die Ausführung der optischen Sensoranordnung als zumindest ein photoelektrischer Sensor, z.B. ein Laser-Line-Scanning-Sensor, sinnvoll, um die Größenerfassung der vereinzelten Körner im Probenkornstrom auch unter weniger idealen Bedingungen durchführen zu können.

Kommt es hingegen zur dauernden Berührung der Körner im Probenkornstrom und überlappen sich die Körner in mehreren Schichten, so kann die optische Sensoranordnung als zumindest eine Kamera mit Blitzlichtbeleuchtung ausgeführt sein. Bei einer Ausführung der optischen Sensoranordnung als zumindest eine Kamera mit Blitzlichtbeleuchtung kann zumindest ein künstliches neuronales Netzwerk zur quantitativen Analyse von aufgenommenen Bilddaten eingesetzt werden.

Vorteilhaft ist es, wenn von der Entnahmevorrichtung abgegebene Körner einem im Wesentlichen senkrecht orientierten hohlzylindrischen Körper, der zumindest teilweise aus Glas oder einem anderen durchsichtigen Material besteht, zugeführt werden. Die Körner können entlang seiner Wandung durch den Körper strömen und/oder sich im freien Fall durch den hohlzylindrischen Körper bewegen. Die optische Sensoranordnung ist benachbart zum hohlzylindrischen Körper angeordnet, sodass diese den Probenkornstrom optisch erfassen kann.

Bevorzugt kann die Auswerteeinheit mit einer externen Datenbank verbindbar sein, an die zumindest eine Handlungsempfehlung für einen nachfolgenden Verarbeitungsprozess des Erntegutes übertragbar ist, welche durch die Auswerteeinheit aus Informationen zur Proteinverschiebung bestimmt wird, wobei die zumindest eine Handlungsanweisung in der externen Datenbank für autorisierte Dritte abrufbar hinterlegt ist.

Bevorzugt kann die Auswerteeinheit dazu ausgeführt und eingerichtet sein, die von der optischen Sensoranordnung empfangenen Signale auszuwerten, um Körner, welche einen vorgebbaren Schwellwert für die Korngröße und/oder einen vorgebbaren Schwellwert für das Korngewicht überschreiten oder unterschreiten, zu identifizieren. Der Schwellwert für die Korngröße und/oder das aus der Korngröße abgeleitete Korngewicht kann insbesondere erntegutabhängig vorgebbar sein. Hierdurch kann für Fruchtarten, die sehr kleine Körner aufweisen, wie sie beispielsweise für Raps, Leinsamen oder Gräser, typisch sind, für Fruchtarten, die sich durch Körner kleiner oder mittlerer Größe auszeichnen, wie beispielswese bei Weizen oder Gerste, und für Fruchtarten, für die große Körner typisch sind, beispielsweise Mais oder Soja, eine Maximalgröße oder eine Minimalgröße bzw. ein Maximalgewicht oder ein Minimalgewicht vorgegeben werden, welche bzw. welches zur Ansteuerung der Abscheidevorrichtung verwendet wird. Dies ermöglich ein optisches Sieben des Probenkornstromes in Abhängigkeit von einer Präferenz hinsichtlich der Inhaltsstoffe, um diese an Anforderungen in einem späteren Weiterverarbeitungsprozess des Erntegutes durch vorbereitende Maßnahmen anpassen zu können.

Die vorliegende Erfindung wird nachstehend anhand eines in den Zeichnungen dargestellten Ausführungsbeispieles näher erläutert.

Es zeigen:
- Fig. 1: schematisch und exemplarisch eine Seitenansicht eines Mähdreschers;
- Fig. 2: schematisch und exemplarisch eine Entnahmevorrichtung einer Probenentnahmevorrichtung;
- Fig. 3: schematisch und exemplarisch die in Bypass-Anordnung am Kornelevator angeordnete Probenentnahmevorrichtung; und
- Fig. 4: ein vereinfachtes Ablaufdiagramm zur Visualisierung von Verfahrensschritten zur Online-Analyse des Erntegutes.

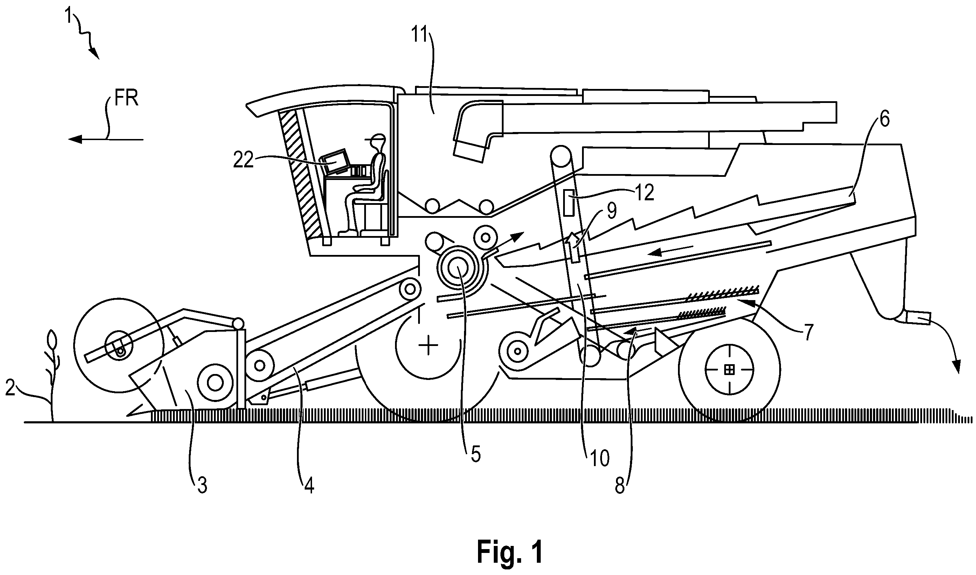

Fig. 1 zeigt schematisch und exemplarisch eine Seitenansicht eines selbstfahrenden Mähdreschers 1 zur Aufnahme und Behandlung von Erntegut 2. Im Erntebetrieb fährt der Mähdrescher 1 in Vorwärtsfahrtrichtung FR durch einen Erntegutbestand 2 und nimmt dabei mit einem erntegutspezifischen Vorsatzgerät 3, beispielsweise einem Schneidwerk oder einem Maispflücker, das Erntegut 2 auf. Mit einem Schrägförderer 4 wird das aufgenommene Erntegut 2 zum Dreschwerk 5 gefördert. In dem Dreschwerk 5, einer Abscheidung 6 und einer Förder- und Reinigungsvorrichtung 7 werden im Erntegut 2 enthaltene Körner 8, 8a vom restlichen Erntegut 2 getrennt, sodass ein aus den Körnern 8, 8a bestehender Erntegutstrom 9 entsteht.

Der Mähdrescher 1 weist einen Kornelevator 10 zur Förderung des aus den Körnern 8, 8a bestehender Erntegutstroms 9 von der Förder- und Reinigungsvorrichtung 7 zu einem Korntank 11 des Mähdreschers 1 auf. An dem Kornelevator 10 ist eine Probenentnahmevorrichtung 12 in Bypass-Anordnung angeordnet. Durch die Probenentnahmevorrichtung 12 wird ein Probenkornstrom 13 des durch den Kornelevator 10 geführten Erntegutstroms 9 aufgenommen. Der Probenkornstrom 13 strömt vom Kornelevator 10 in die Probenentnahmevorrichtung 12 ein und durchströmt diese. Beim Verlassen der Probenentnahmevorrichtung 12 wird der Probenkornstrom 13 wieder zurück in den Kornelevator 10 geführt.

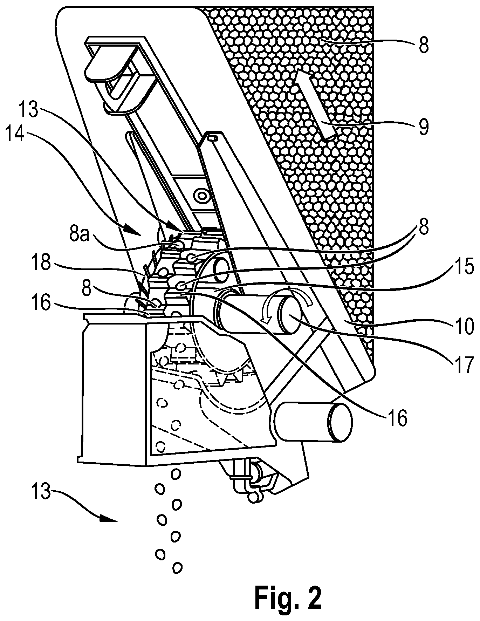

Die Darstellung in Fig. 2 zeigt schematisch und exemplarisch eine rotierend angetriebene Entnahmevorrichtung 14 der in Bypass-Anordnung am Kornelevator 10 angeordneten Probenentnahmevorrichtung 12. Im dargestellten Ausführungsbeispiel ist die Entnahmevorrichtung 14 an der in Fahrtrichtung FR weisenden Seite des Kornelevators 10 angeordnet. Der Kornelevator 10 weist eine im Wesentlichen konvexe Ausbuchtung mit einer Öffnung auf, durch welche die Entnahmevorrichtung 14 abschnittsweise in den durch den Kornelevator 10 geführten Erntegutstrom 9 eingreift. Die im Wesentlichen konvexe Ausbuchtung bildet eine Senke in der Wandung des Kornelevators 10, welcher die Körner 8 im Erntegutstrom 9 kontinuierlich zugeführt werden. Der Entnahmevorrichtung 14 werden somit permanent Körner 8, 8a zur Entnahme zugeführt.

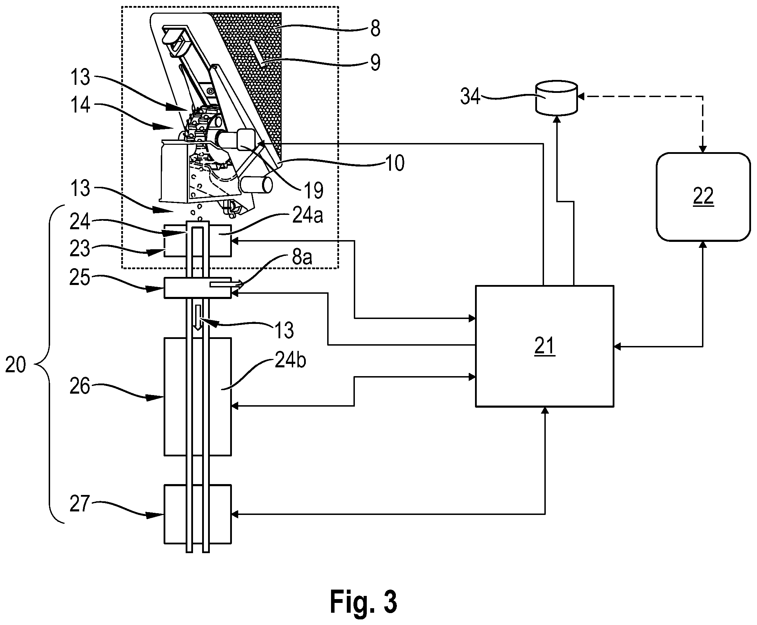

Die Entnahmevorrichtung 14 ist gemäß der in Fig. 2 dargestellten Ausführungsform als zumindest ein rotierend angetriebenes Nockenrad 15 mit zumindest einer umlaufenden Nockenreihe 16, hier und vorzugsweise mit zwei Nockenreihen 15, ausgeführt. Das Nockenrad 15 ist auf einer angetriebenen Welle 17 drehfest angeordnet. Zum Antreiben der Welle 17 kann ein Zahnrad 18 drehfest auf der Welle 17 angeordnet sein, welches mit einem durch einen Aktuator angetriebenes Zahnrad kämmt. Alternativ kann die Welle 17 direkt durch einen Aktuator antreibbar sein, wie in Fig. 3 beispielhaft angedeutet.

Bevorzugt können zwei oder mehr Nockenräder 15 koaxial nebeneinander auf der Welle 17 angeordnet sein, wobei die zumindest eine umlaufende Nockenreihe 16 des jeweiligen Nockenrades 15 einen Versatz in Umfangsrichtung aufweist. Alternativ kann ein Nockenrad 15 mit zwei oder mehr in Umfangsrichtung zueinander versetzt angeordneten Nockenreihen 16 vorgesehen sein, wie in Fig. 2 beispielhaft dargestellt.

Mittels der rotierend angetriebenen Entnahmevorrichtung 14 werden zur kontinuierlichen Entnahme des Probenkornstroms 13 einzelne Körner 8, 8a aus dem Erntegutstrom 9 separiert. Das zumindest eine Nockenrad 15 oder das Zellenrad wird bevorzugt mit einer Drehzahl angetrieben, um einen Massenstrom im Bereich zwischen 10 g/s und 100 g/s, vorzugsweise im Bereich zwischen 20 g/s und 50 g/s, bereitzustellen, mit dem der Probenkornstrom 13 der optischen Sensoranordnung 23 zugeführt wird. Bei Massenströmen im bevorzugten Bereich zwischen 20 g/s und 50 g/s strömen die entnommenen Körner 8, 8a im Wesentlichen in einer eindimensionalen Schicht an der optischen Sensoranordnung 23 vorbei. Als eindimensionale Schicht ist die Verteilung der Körner 8, 8a nebeneinander über eine Fläche bezeichnet, wobei es im Idealfall in vertikaler Richtung nur eine einzelne Lage Körner 8, 8a gibt.

Der von der Entnahmevorrichtung 14 abgegebene Probenkornstrom 13 wird einer der Entnahmevorrichtung 14 nachgeordneten optischen Sensoranordnung 23 zur Größenerfassung der vereinzelten Körner 8, 8a im Probenkornstrom 13 zugeführt. Hierzu ist es vorteilhaft, wenn die von der Entnahmevorrichtung 14 abgegebenen Körner 8, 8a einem senkrecht oder im Wesentlichen senkrecht orientierten hohlzylindrischen Körper 24, der zumindest teilweise aus Glas oder einem anderen durchsichtigen Material besteht, zugeführt werden. Die optische Sensoranordnung 23 ist benachbart zum Körper 24 angeordnet, sodass diese den Probenkornstrom 13 im Körper 24 optisch erfassen kann. Bevorzugt kann die Anzahl der hohlzylindrischen Körper 24, denen die Körner 8 des Probenkornstroms 13 zugeführt werden, der Anzahl der Nockenrädern 15 entsprechen, welche die Entnahmevorrichtung 14 umfasst. Dabei kann zu jedem hohlzylindrischen Körper 24 benachbart eine eigene optische Sensoranordnung 23 angeordnet sein.

In Fig. 3 ist schematisch und exemplarisch die in Bypass-Anordnung am Kornelevator 10 angeordnete Probenentnahmevorrichtung 12 dargestellt. Die Probenentnahmevorrichtung 12 weist eine Messanordnung 20 mit einer Auswerteeinheit 21 auf. Die Auswerteeinheit 21 kann mit einer Benutzerschnittstelle 22 in der Kabine des Mähdreschers 1 verbunden sein. Bevorzugt umfasst die Benutzerschnittstelle 22 eine Eingabe-AusgabeEinheit. Alternativ oder zusätzlich kann die Auswerteeinheit 21 mit einer externen Datenverarbeitungsvorrichtung verbindbar sein, um generierte Daten an diese zur weiteren Verwendung zu übertragen.

Die der Entnahmevorrichtung 14 nachgeordnete optische Sensoranordnung 23 zur Größenerfassung der vereinzelten Körner 8, 8a im Probenkornstrom 13 überträgt ihre Signale an die Auswerteeinheit 21 zur Auswertung. Die Auswerteeinheit 21 ist dazu eingerichtet und programmiert, mittels der von der optischen Sensoranordnung 23 bereitgestellten Signalen, einzelne Körner 8, 8a hinsichtlich ihrer Größe zu unterscheiden. Dabei werden die von der optischen Sensoranordnung 23 erfassten und optisch vermessenen Körner 8, 8a in unterschiedliche Gewichtsklassen eingeteilt. Entsprechend der jeweilig bestimmten Größe eines Korns 8, 8a wird dabei zumindest näherungsweise auf dessen jeweiliges Korngewicht geschlossen, um das Korn 8, 8a einer der Gewichtsklassen zuzuordnen.

Die Auswerteeinheit 21 ist dazu ausgeführt und programmiert, die von der optischen Sensoranordnung 23 empfangenen Signale auszuwerten, um einzelne Körner 8a im Probenkornstrom 13, welche einen erntegutabhängig vorgebbaren Schwellwert für eine Korngröße und/oder einen erntegutabhängig vorgebbaren Schwellwert für ein Korngewicht überschreiten oder unterschreiten, zu identifizieren und einer gemeinsamen Gewichtsklasse zuzuordnen. Der erntegutspezifische Schwellwert kann einem Maximalwert oder einem Minimalwert für die Korngröße entsprechen.

Die von der Entnahmevorrichtung 14 der nachgeordneten optischen Sensoranordnung 23 zugeführten Körner 8, 8a des Probenkornstroms 13 werden von der optischen Sensoranordnung 23 erfasst und optisch vermessen. Die optisch erfassten und vermessenen Körner 8, 8a werden, mit Ausnahme der Körner 8a, bei der Auswertung der Signale durch die Auswerteeinheit 21 mittels eines Algorithmus in zumindest sechs unterschiedliche Gewichtsklassen eingeteilt. Die Anzahl von zumindest sechs Gewichtsklassen dient der Annäherung der erwartbaren Verteilung der Korngrößen respektive der Verteilung des daraus abgeleiteten Korngewichts bezogen auf den vom Kornelevator 10 geförderten Massedurchsatz.

Wie bereits ausgeführt, werden in eine der zumindest sechs Gewichtsklassen alle erfassten Körner 8a einsortiert, welche den zumindest einen erntegutabhängig vorgebbaren Schwellwert überschreiten oder unterschreiten. Die übrigen Gewichtsklassen weisen bevorzugt Gewichtsintervalle mit im Wesentlichen gleicher Größe auf. Hierdurch kann zumindest näherungsweise auf eine Gewichtsverteilung der Körner 8 in dem vom Kornelevator 10 geförderten Erntegutstrom 9 geschlossen werden.

Der hohlzylindrische Körper 24 kann in mehrere Segmente 24a, 24b unterteilt sein, die entlang der Messanordnung 20 angeordnet sind. Die einzelnen Segmente 24a, 24b werden zumindest abschnittsweise vorzugsweise aus Borosilikatglas, auch "technisches Glas" genannt, hergestellt. Borosilikatglas ist robust und kostengünstig. Zudem ist Borosilikatglas sowohl im sichtbaren Wellenlängenbereich als auch im größten Teil des NIR-Wellenlängenbereichs transparent.

Die optischen Sensoranordnung 23 umfasst zumindest eine Lichtschranke, z.B. LED-Einweglichtschranke, und/oder zumindest einen Reflexionssensor, z.B. LED-Reflexionssensor, und/oder zumindest einen photoelektrischen Sensor, z.B. Laser-Line-Scanning-Sensor. Die mögliche Ausführung der optischen Sensoranordnung 23 hängt dabei von der Gleichförmigkeit des Probenkornstroms 13 und dessen Schichtdicke ab. Besteht der Probenkornstrom 13 im Wesentlichen aus einer einzelnen Schicht aus Körnern 8, 8a, die mit im Wesentlichen gleichmäßigem Abstand zueinander die optische Sensoranordnung 23 passieren, kann die optische Sensoranordnung z.B. als zumindest eine LED-Einweglichtschrank oder als zumindest ein LED- Reflexionssensor ausgeführt sein. In Strömungssituationen, in denen die Schichtdicke des Probenkornstroms 13 ungleichmäßiger und/oder die Abstände zwischen den einzelnen Körnern 8, 8a im Probenkornstrom 13 ungleichmäßig und/oder zu gering sind, d.h. es insbesondere zu Berührungen kommt, ist die Ausführung der optischen Sensoranordnung 23 als zumindest ein photoelektrischer Sensor, z.B. ein Laser-Line-Scanning-Sensor, sinnvoll, um die Größenerfassung der vereinzelten Körner 8, 8a im Probenkornstrom 13 auch unter diesen weniger idealen Bedingungen durchführen zu können.

Die Probenentnahmevorrichtung 12 weist eine der optischen Sensoranordnung 23 optional nachgeordnete Abscheidevorrichtung 25 auf. Die Abscheidevorrichtung 25 ist dazu ausgeführt, in Abhängigkeit von den durch die Auswerteeinheit 21 ausgewerteten, von der optischen Sensoranordnung 23 zur ausgeführten Größenunterscheidung respektive Gewichtsbestimmung bereitgestellten Signalen, einzelne Körner 8a aus dem Probenkornstrom 13 abzuscheiden. Hierbei wurden die im Probenkornstrom 13 enthaltenen Körner 8 durch die optische Sensoranordnung 23 als abzuscheidende Körner 8a des Probenkornstroms 13 identifiziert, welche den vorgebbaren erntegutspezifischen Schwellwert überschreiten oder unterschreiten.

Insbesondere kann die Abscheidevorrichtung 25 zur pneumatischen Abscheidung der einzelnen Körner 8a aus dem Probenkornstrom 13 ausgeführt und eingerichtet sein. Durch die Vereinzelung der Körner 8 mittels der Entnahmevorrichtung 14 sowie einem optionalen, der Entnahmevorrichtung 14 nachgeordneten mechanischen Vereinzelungselement kann die Abscheidevorrichtung 25 aufgrund ihrer Größe auszusortierende Körner 8a durch gezielte Luftstöße aus dem Probenkornstrom 13 abscheiden. Bevorzugt können die aussortierten und abgeschiedenen Körner 8a dem Kornelevator 10 wieder zugeführt werden.

Zur Abscheidung der Körner 8a sind die zumindest zwei Segmente 24a, 24b des Körpers 24 im Bereich der Abscheidevorrichtung 25 zueinander beabstandet, so dass sich ein offener Übergangsbereich zwischen diesen ausbildet. In dem offenen Übergangsbereich können die aufgrund ihrer Größe abzuscheidenden Körner 8a vom übrigen Probenkornstrom 13 durch gezielte Luftstöße, die von der Abscheidevorrichtung 25 abgegeben werden, nach außen abgeschieden werden. Zur Ansteuerung der Abscheidevorrichtung 25 ist die Auswerteeinheit 21 mit dieser signaltechnisch verbunden.

Die Probenentnahmevorrichtung 12 umfasst weiterhin einen der Abscheidevorrichtung 25 nachgeordneten optischen Sensor 27, der zur Bestimmung der Inhaltsstoffe der im Probenkornstrom 13 verbliebenen Körner 8 ausgeführt und eingerichtet ist. Der optische Sensor 27, welcher der Abscheidevorrichtung 25 nachgeordnet angeordnet ist, ist als NIR-Sensor ausgeführt. Mittels des als NIR-Sensor ausgeführten Sensors 27 werden die Inhaltsstoffe in den Körnern 8 bestimmt. Bei dem zu analysierenden Erntegut handelt es sich insbesondere um Getreide, aber auch Ölfrüchte oder Leguminosen kommen als Erntegut infrage. Zu den zumindest qualitativ zu bestimmenden Inhaltsstoffen des Korns 8 gehören Kohlenhydrate, Proteine, Feuchte (Wassergehalt), Öle.

Gemäß einer alternativen Ausführung der optionalen Abscheidevorrichtung 25 kann diese als ein mechanisches Sieb ausgeführt sein, welches entsprechend des Schwellwertes für eine maximale oder minimale Korngröße die Körner 8a aus dem Probenkornstrom 13 abscheidet, so dass nur die Körner 8 dem optischen Sensor 27 zugeführt werden, die diesen erntegutspezifischen Schwellwert unterschreiten bzw. überschreiten.

Dem optischen Sensor 27 kann eine optionale Sensoreinheit 26 vor- oder nachgeschaltet sein. Die optionale Sensoreinheit 26 kann beispielsweise zwischen der Abscheidevorrichtung 25 und dem optischen Sensor 27 positioniert sein. Die Sensoreinheit 26 umfasst einen Sensor oder mehrere Sensoren unterschiedlicher Sensortechnologien, die zur Bestimmung von Parametern eingerichtet sind, welche Rückschlüsse auf das Vorliegen von Bruchkorn, Korngesundheit, Besatz, Oberflächenbeschaffenheit des Korns und/oder Farbe des Korns 8 im Probenkornstrom 13 ermöglichen.

Die Messanordnung 20 weist somit die optische Sensoranordnung 23, die optionale Abscheidevorrichtung 25, die optionale Sensoreinheit 26 und den optischen Sensor 27 auf, welche mit der Auswerteeinheit 21 drahtlos oder drahtgebunden im Datenaustausch stehen.

Durch die mittels des als NIR-Sensor ausgeführten Sensors 27 erfassten und durch die Auswerteeinheit 21 bestimmen Absolut- und/oder Relativwerte für die Inhaltsstoffe Kohlenhydrate, Proteine, Feuchte, Öle und die mittels der optischen Sensoranordnung 23 erfassten Größen der Körner 8, 8a und deren Auswertung durch die Auswerteeinheit 21 lässt sich auf den Proteingehalt in Relation zur Körnergröße schließen.

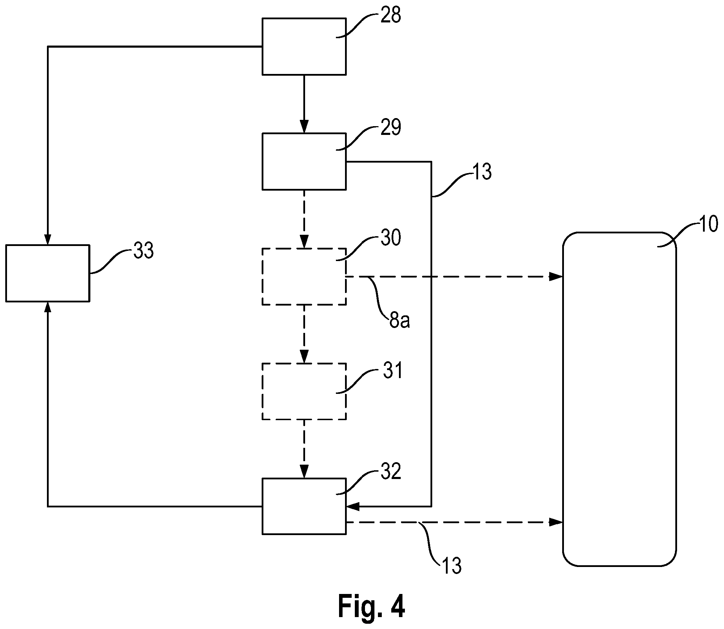

In Fig. 4 ist ein vereinfachtes Ablaufdiagramm dargestellt, welches die notwendigen und optionalen Verfahrensschritte veranschaulicht, die zur Bestimmung der Information zur Proteinverschiebung PSI während des Erntevorganges durch den Mähdrescher 1 durchgeführt werden. Wesentlich ist, dass die Probenentnahmevorrichtung 12 und das Verfahren zur Bestimmung der Information zur Proteinverschiebung PSI im laufenden Betrieb des Mähdrescher 1 betrieben werden.

Im Verfahrensschritt 28 erfolgt das Separieren von einzelnen Körnern 8 aus dem Erntegutstrom 9 im Elevator 10, um den Probenkornstrom 13 zu erzeugen. Hierzu werden durch die rotierend angetriebene Entnahmevorrichtung 14 kontinuierlich einzelne Körner 8, 8a als Probenkornstrom 13 separiert, wobei der Probenkornstrom 13 einen im Wesentlichen konstanten Massenstrom vorgebbarer Größe aufweist.

Im Verfahrensschritt 29 wird die Größe der Körner 8, 8a im Probenkornstrom 13 mittels der optischen Sensoranordnung 23 optisch vermessen, um die Körner 8, 8a den zumindest sechs unterschiedlichen Gewichtskassen zuzuordnen. Die Auswertung der Signale der optischen Sensoranordnung 23 zur optischen Vermessung erfolgt im Fall einer kamerabasierten Sensoranordnung 23 mittels eines Bildauswertungsalgorithmus durch die Auswerteeinheit 21. Dabei wird auch die Anzahl der einer jeweiligen Gewichtsklasse zugeordneten Körner 8, 8a bestimmt und in einer Speichereinheit der Auswerteeinheit 21 hinterlegt. Der bevorzugte Massenstrom für den entnommenen Probenkornstrom 13 liegt vorzugsweise zwischen 10 g/s und 100 g/s, äußerst bevorzugt zwischen 20 g/s und 50 g/s.

Innerhalb eines der Probenentnahmevorrichtung 12 vorgebbaren Messzeitraumes werden eine vorbestimmte Anzahl an aufeinanderfolgenden Messungen mit einem vorgebbaren Messintervall zur Bestimmung der Korngröße durchgeführt. Die Ergebnisse dieser Messungen werden den zumindest sechs unterschiedlichen Gewichtsklassen zugeordnet, so dass sich mittels eines Softwarealgorithmus ein Histogramm der Korngrößenverteilung im Probenkornstrom 13 durch die Auswerteeinheit 21 erstellen lässt. Die bekannte bzw. einstellbare Größenordnung des Massenstroms des entnommenen Probenkornstroms 13 führt zu einer bekannten und nahezu konstanten Gesamtanzahl von Körnern 8, 8a pro Messintervall, welche innerhalb des Messintervalls von der optischen Sensoranordnung 23 optisch vermessen werden.

Das mittels der Probenentnahmevorrichtung 12 durchführbare Verfahren kann, wie durch die strichlinierte Darstellung in Fig. 4 angedeutet, zumindest einen weiteren, jeweils optionalen Verfahrensschritt 30 und/oder Verfahrensschritt 31 umfassen, was weiter unten näher erläutert wird.

Zunächst wird das weitere Verfahren ohne den zumindest einen optionalen Verfahrensschritt 30 und/oder den optionalen Verfahrensschritt 31 beschrieben.

Dabei wird der Probenkornstrom 13 nach der optischen Vermessung durch die optische Sensoranordnung 23 vollständig, d.h. ohne eine Abscheidung der Körner 8a, dem als NIR-Sensor ausgeführten Sensor 27 zur Inhaltsstoffanalyse zugeführt. Mittels des NIR-Sensors werden im Idealfall alle Körner 8, 8a im Probenkornstrom 13 hinsichtlich ihrer Inhaltsstoffe detektiert, die innerhalb des vorgegebenen Messintervalls auch die optische Sensoranordnung 23 passiert haben.

Im Idealfall sind die Messintervalle der optischen Sensoranordnung 23 und des als NIR-Sensor ausgeführten Sensors 27 zur Inhaltsstoffanalyse exakt aufeinander abgestimmt, um bei der nachfolgenden Bestimmung der Information zu Protein-Verschiebung PSI durch die Auswerteeinheit 21 eine möglichst hohe Genauigkeit zu erreichen.

Im Verfahrensschritt 32 werden die Signale, welche die Inhaltsstoffe aller Körner 8, 8a des Probenkornstrom 13 repräsentieren, durch den als NIR-Sensor ausgeführten Sensor 27 generiert und an die Auswerteeinheit 21 zur Bestimmung der Inhaltsstoffe übermittelt.

Die bestimmten Inhaltsstoffe umfassen, wie weiter oben bereits ausgeführt, zumindest den Proteingehalt, den Ölgehalt und den Feuchtegehalt, die aufgrund der Detektion mittels des NIR-Sensors als Relativwerte bezogen auf die Trockenmasse bzw. die Gesamtmasse der Körner 8, 8a bestimmt werden.

Nachdem der Probenkornstrom 13 den Sensor 27 zur Inhaltsstoffanalyse passiert hat, wird der Probenkornstrom 13 dem Kornelevator 10 wieder zugeführt.

Im Verfahrensschritt 33 werden die von der optischen Sensoranordnung 23 und dem als NIR-Sensor ausgeführten Sensor 27 bereitgestellten Signale von der Auswerteeinheit 21 verarbeitet. Der Auswertung liegen dabei die Histogramme der Verteilung der Korngrößen bezogen auf die zumindest sechs unterschiedlichen Gewichtsklassen vor. Zudem ist der Gesamtproteingehalt des Probenkornstroms 13 bezogen auf die Trockenmasse des Probenkornstroms 13 bekannt, welcher den Sensor 27 zur Inhaltsstoffanalyse innerhalb des jeweiligen Messintervalls passiert. Aus diesen bekannten Informationen wird durch die Auswerteeinheit 21 die Information zur Proteinverschiebung PSI, welche den Proteingehalt der Körner 8, 8a innerhalb einer jeweiligen der zumindest sechs Gewichtsklassen darstellt, bestimmt.

Nachstehend ist beispielhaft eine Formel angegeben, welche den vorstehen beschriebenen Zusammenhang für zumindest sechs Gewichtsklassen wiedergibt: $\left(N{\left(t\right)}_{\left(mx6\right)}\times {\overline{m}}_{\left(6x6\right)}\right) \times {P%}_{\left(6x1\right)} = mP {\left(t\right)}_{\left(mx1\right)}$ (1) $mit N {\left(t\right)}_{\left(mx6\right)} = \left⌈\begin{matrix}{n}_{1 , 1} & {n}_{2 , 1} & {n}_{3 , 1} & {n}_{4 , 1} & {n}_{5 , 1} & {n}_{6 , 1} \\ {n}_{1 , 2} & {n}_{2 , 2} & {n}_{3 , 1} & {n}_{4 , 2} & {n}_{5 , 2} & {n}_{6 , 2} \\ {n}_{1 , 3} & {n}_{2 , 3} & {n}_{3 , 3} & {n}_{4 , 3} & {n}_{5 , 3} & {n}_{6 , 3} \\ ⋮ & ⋮ & ⋮ & ⋮ & ⋮ & ⋮ \\ {n}_{1 , m - 1} & {n}_{2 , m - 1} & {n}_{3 , m - 1} & {n}_{4 , m - 1} & {n}_{5 , m - 1} & {n}_{6 , m - 1} \\ {n}_{1 , m} & {n}_{2 , m} & {n}_{3 , m} & {n}_{4 , m} & {n}_{5 , m} & {n}_{6 , m}\end{matrix}\right⌉$ ${\overline{m}}_{\left(6x6\right)} = \left⌈\begin{matrix}\overline{m} 1 & 0 & 0 & 0 & 0 & 0 \\ 0 & \overline{m} 2 & 0 & 0 & 0 & 0 \\ 0 & 0 & \overline{m} 3 & 0 & 0 & 0 \\ 0 & 0 & 0 & \overline{m} 4 & 0 & 0 \\ 0 & 0 & 0 & 0 & \overline{m} 5 & 0 \\ 0 & 0 & 0 & 0 & 0 & \overline{m} 6\end{matrix}\right⌉$ ${P%}_{\left(6x1\right)} = \left⌈\begin{matrix}P% 1 \\ P% 2 \\ P% 3 \\ P% 4 \\ P% 5 \\ P% 6\end{matrix}\right⌉$ und $mP {\left(t\right)}_{\left(mx1\right)} = \left⌈\begin{matrix}mP \left(t-\left(m-1\right)s\right) \\ mP \left(t-\left(m-2\right)s\right) \\ mP \left(t-\left(m-3\right)s\right) \\ ⋮ \\ mP \left(t-1s\right) \\ mP \left(t\right)\end{matrix}\right⌉ .$

Die Matrix *N*(*t*) enthält die Anzahl *n* der Körner 8, 8a je Messung, die auf eine der zumindest sechs Gewichtsklassen verteilt sind. Die Diagonalmatrix *m̅* enthält vorgegebene Werte, die das durchschnittliche Korngewicht jeder der zumindest sechs Gewichtsklasse beschreiben. Mit *m* ist die Anzahl der Messungen innerhalb eines Messzeitraumes bezeichnet. Der Vektor *mP*(*t*) enthält die Proteinmassen, die vom Sensor 27 zur Inhaltsstoffanalyse innerhalb der jeweiligen Messintervalle für alle im Probenkornstroms 13 enthaltenen Körner 8, 8a gemeinsam gemessen wurden. Der Vektor P% enthält die zu bestimmende Konzentration an Proteingehalt innerhalb jeweils einer der zumindest sechs Gewichtsklassen. Die Kenntnis über die Verteilung der Konzentration des Proteingehalts auf die jeweilige Gewichtsklasse, ermöglicht es, das Resultat einer nachfolgenden Siebung des gesamten geernteten Korns 8, 8a, das in zumindest einem befüllten Korntank 11 enthalten ist, zu bestimmen, was als Information zur Proteinverschiebung PSI bezeichnet wird. Die Gleichung (1) ist eine lineare Regressionsgleichung, die für den Vektor P% durch die Auswerteeinheit 21 mittels linearer Regressionsanalyse gelöst werden kann.

Der Betreiber des Mähdrescher 1 kann auf Basis dieser Information zur Proteinverschiebung PSI entscheiden, ob er auf eigenen Kosten eine Siebung durchführt, um den Proteingehalt im geernteten Korn 8, 8a zum Beispiel zu erhöhen und damit einen höheren Ertrag beim Verkauf des Korns 8, 8a an eine Getreidemühle oder einen Zwischenhändler zu erreichen. Indem die Körner 8a ausgesiebt werden, deren Korngröße oberhalb des Schwellwertes liegt und damit deren Proteingehalt in Relation zur Korngröße geringer ist als beim übrigen kleineren Korn 8, kann der Gesamtproteingehalt angehoben werden, um beispielsweise das Qualitätskriterium für Brotweizen zu erfüllen. Umgekehrt kann z.B. im Fall von Braugerste als Erntegut der Proteingehalt einer Charge unterhalb eines vom Markt gewünschten Maximalwertes gesenkt werden, indem tendenziell kleinere Körner 8a, deren Gewichtsklassen einen tendenziell höheren Proteingehalt aufweisen, aussortiert werden.

Der optionale Verfahrensschritt 30, welcher die gezielte Abscheidung von einzelnen Körnern 8a aus dem Probenkornstrom 13 mittels der Abscheidevorrichtung 25 zum Gegenstand hat, kann in Ergänzung zur Modifikation der vorstehend beschriebenen Verfahrensschritte 29 und 32 zur Anwendung kommen.

Dem liegt die Überlegung zugrunde, dass während der durchzuführenden Messungen durch die optische Sensoranordnung 23 und den Sensor 27 zur Inhaltsstoffanalyse bei der Erfassung des Korns 8, 8a im Probenkornstrom 13 eine zeitliche Diskrepanz in einer Größenordnung auftreten kann, die dazu führt, dass die optische Sensoranordnung 23 andere Körner 8, 8a erfasst als der Sensor 27 zur Inhaltsstoffanalyse im nahezu identischen Zeitpunkt des Messintervalls. Die zeitliche Diskrepanz führt dazu, dass die Genauigkeit der Bestimmung der Information zur Proteinverschiebung PSI abnimmt, da die von der Auswerteeinheit 21 durchgeführte lineare Regressionsanalyse zur Lösung der Gleichung (1) im Ergebnis lediglich zu einer Annäherung führt, die für eine ausreichend genaue Bestimmung der Information zur Proteinverschiebung PSI als eine Handlungsempfehlung unzureichend ist.

Mittels der Abscheidevorrichtung 25 werden die Körner 8a, deren Größe den vorgegebenen erntegutspezifischen Schwellwert überschreiten oder unterschreiten, abgeschieden, bevor diese den Sensor 27 zur Inhaltsstoffanalyse erreichen. Auf diese Weise lassen sich die Ergebnisse beider Szenarien eines späteren Siebvorgangs, d.h. die Erhöhung des Proteingehalts durch das Aussortieren der großen Körner 8a im Fall von Weizen oder die Verringerung des Proteingehalts durch das Ausscheiden der kleinen Körner 8a im Fall von Braugerste, vorherbestimmen.

Durch die Bestimmung der Anzahl von Körnern 8a durch die optische Sensoranordnung 23, welche, beispielsweise im Fall von Weizen, den Schwellwert überschreiten, und deren daraus bestimmten prozentualem Anteil an der Gesamtmasse des Probekornstroms 13, entfällt die Inhaltsstoffbestimmung durch den Sensor 27 zur Inhaltsstoffanalyse für diese sechste Gewichtsklasse.

Die Gleichung (1) berücksichtigt daher nur die zumindest fünf Gewichtsklassen der Körner 8, deren Anzahl je Gewichtsklasse bestimmt wird sowie für welche die Proteinmasse des um die Körner 8a reduzierten Probenkornstroms 13 durch den Sensor 27 zur Inhaltsstoffanalyse bestimmt wird. Im Übrigen wird die Gleichung in identischer Weise von der Auswerteeinheit 21 mittels linearer Regressionsanalyse gelöst, um den Vektor P% zu bestimmen. Insbesondere kann unter Verwendung von Vektor P% die Information zur Proteinverschiebbarkeit (PSI) während einer Erntekampagne auf einem Feld über mehrere Korntankfüllungen hinweg akkurat aufintegriert werden, so dass die Information zur Proteinverschiebbarkeit (PSI) des Gesamterntegutes am Ende akkurat bestimmt wird. Hierzu kann der z.B. sekündlich aktualisierte Vektor P% gewichtet mit dem aktuellen Ausgangssignal (Tonnen/Std) eines Ertragssensors, welcher auf Mähdreschern in der Regel vorhanden ist, und typisch ebenfalls mit einer Zeitauflösung in der Größenordnung von 1 s messen. Beide Signale können auch erst über mehrere Sekunden gemittelt werden (Tiefpassfilterung), bevorzugt über 10 s bis 30 s, und die gewichtete Integration dann mit den gemittelten Daten durchgeführt werden.

Bei einem Vorhandensein der optionalen Abscheidevorrichtung 25 kann auf die Berechnung des Vektors P%, d.h. auf das periodische Ausrechnen von Gleichung (1), alternativ auch verzichtet werden, da aus der Messung der Inhaltsstoffe des optisch gesiebten Probenkornstrom 13, d.h. ohne die abgeschiedenen Körner 8a, der Effekt einer eventuellen späteren Siebung des Erntegutes direkt ermittelt ist und näherungsweise auch aufintegriert werden kann.

Der ebenfalls optionale Verfahrensschritt 31, der mittels der Sensoreinheit 26 umgesetzt wird, dient, wie weiter oben bereits ausgeführt, dazu zusätzliche Parameter des im Probenkornstrom 13 enthaltenen Korns 8, 8a zu bestimmen, welche Rückschlüsse auf das Vorliegen von Bruchkorn, Korngesundheit, Besatz, Oberflächenbeschaffenheit des Korns und/oder Farbe des Korns 8, 8a ermöglichen. Dies sind zusätzlich generierbare Informationen für den Betreiber des Mähdreschers 1, welche ergänzende Informationen zur Qualität des im Korntank 11 befindlichen Erntegutes und damit des zu erwartenden Verkaufserlöses darstellen.

Die zumindest eine von der Auswerteeinheit 21 generierte Handlungsempfehlung wird an eine entfernte Datenbank 34 übertragen, in welcher die zumindest eine Handlungsanweisung für autorisierte Dritte abrufbar hinterlegt ist. Der Betreiber des selbstfahrenden Mähdreschers 1 oder der Eigentümer des geernteten Korns 8, 8a können durch eine Registrierung den Zugriff auf die zumindest eine Handlungsempfehlung erhalten, welche mittels des Verfahrens aufgrund der bestimmten Informationen zur Proteinverschiebung PSI generiert wird. Neben dem Abruf der zumindest einen Handlungsempfehlung kann vorgesehen sein, dass die mittels der optionalen Sensoreinheit 26 bestimmten Parameter zum Vorhandensein von Bruchkorn, der Korngesundheit, dem Besatz, der Oberflächenbeschaffenheit des Korns 8, 8a und/oder der Farbe des Korns 8, 8a im Probenkornstrom 13 abgerufen werden können. Mit Bezug auf das Korn 8a gilt dies nur insoweit, als dass keine Abscheidung durch die Abscheidevorrichtung 25 vorgesehen ist.

### Bezugszeichenliste

- 1: Mähdrescher
- 2: Erntegut
- 3: Vorsatzgerät
- 4: Schrägförderer
- 5: Dreschwerk
- 6: Abscheidung
- 7: Förder- und Reinigungsvorrichtung
- 8: Korn
- 8a: Korn
- 9: Erntegutstrom
- 10: Kornelevator
- 11: Korntank
- 12: Probenentnahmevorrichtung
- 13: Probenkornstrom
- 14: Entnahmevorrichtung
- 15: Nockenrad
- 16: Nockenreihe
- 17: Welle
- 18: Zahnrad
- 19: Aktuator
- 20: Messanordnung
- 21: Auswerteeinheit
- 22: Benutzerschnittstelle
- 23: Sensoranordnung
- 24: Körper
- 25: Abscheidevorrichtung
- 26: Sensoreinheit
- 27: Sensor
- 28-33: Verfahrensschritt
- 34: Datenbank

## Patentansprüche

1. Verfahren zur Online-Analyse von Erntegut (2), welches von einem selbstfahrenden Mähdrescher (1) aufgenommen und behandelt wird, wobei ein Erntegutstrom (9) von einem Kornelevator (10) von einer Förder- und Reinigungsvorrichtung (4) zu einem Korntank (5) des Mähdreschers (1) gefördert wird, wobei mit einer am Kornelevator (10) in Bypass-Anordnung angeordneten Probenentnahmevorrichtung (12) ein Probenkornstrom (13) aus dem durch den Kornelevator (10) geführten Erntegutstrom (9) entnommen und Ernteguteigenschaften der im Probenkornstrom (13) enthaltenen Körner (8, 8a) von einer Messanordnung (20) der Probenentnahmevorrichtung (12) mit einer Auswerteeinheit (21) ermittelt werden, **gekennzeichnet durch die Verfahrensschritte:**
- Separierung einzelner Körner (8, 8a) als Probenkornstrom (23) aus dem Erntegutstrom (9) mittels eine Entnahmevorrichtung (14),
- Erfassung von Korngrößen der Körner (8, 8a) im Probenkornstrom (13) durch eine der Entnahmevorrichtung (14) nachgeordnete optische Sensoranordnung (23) und Einteilung in mehrere Gewichtsklassen, und
- Bestimmung von Inhaltsstoffen des vorbeiströmenden Probenkornstromes (13) durch einen optischen Sensor (27), wobei die vom optischen Sensor (27) generierten, den erfassten Inhaltsstoffen entsprechenden Probenkornstromdaten an die Auswerteeinheit (21) übertragen werden, um daraus Informationen zur Proteinverschiebung (PSI) zu bestimmen, welche durch die Auswerteeinheit (21) zur Generierung zumindest einer Handlungsempfehlung für einen nachfolgenden Verarbeitungsprozess des Erntegutes (2) ausgewertet werden.

2. Verfahren nach Anspruch 1, **dadurch gekennzeichnet, dass** als zumindest eine Handlungsempfehlung für den nachfolgenden Verarbeitungsprozess ein Vorschlag für eine Fraktionierung des Erntegutes (2) generiert wird.

3. Verfahren nach Anspruch 1 oder 2, **dadurch gekennzeichnet, dass** die Fraktionierung in Abhängigkeit von den bestimmten Inhaltsstoffanteilen einer jeweiligen Gewichtsklasse durchgeführt wird.

4. Verfahren nach einem der Ansprüche 1 bis 3, **dadurch gekennzeichnet, dass** die zumindest eine generierte Handlungsempfehlung an eine entfernte Datenbank (34) übertragen wird, in welcher die zumindest eine Handlungsanweisung für autorisierte Dritte abrufbar hinterlegt ist.

5. Verfahren nach einem der vorangehenden Ansprüche, **dadurch gekennzeichnet, dass** die von der optischen Sensoranordnung (23) erfassten Körner (8, 8a) auf zumindest sechs Gewichtsklassen verteilt werden.

6. Verfahren nach einem der vorangehenden Ansprüche, **dadurch gekennzeichnet, dass** einzelne Körner (8a), welche einen erntegutabhängig vorgebbaren Schwellwert für eine Korngröße und/oder einen erntegutabhängig vorgebbaren Schwellwert für ein Korngewicht überschreiten oder unterschreiten, identifiziert und einer gemeinsamen Gewichtsklasse zugeordnet werden.

7. Verfahren nach einem der vorangehenden Ansprüche, **dadurch gekennzeichnet, dass** innerhalb eines vorgebaren Messzeitraumes eine vorbestimmte Anzahl an aufeinanderfolgenden Messungen mit einem vorgebbaren Messintervall zur Bestimmung der Korngröße durchgeführt wird, wobei mittels eines Softwarealgorithmus ein Histogramm der Korngrößenverteilung im Probenkornstrom (13) durch die Auswerteeinheit (21) erstellt wird.

8. Verfahren nach Anspruch 7, **dadurch gekennzeichnet, dass** zur Bestimmung der Information zur Proteinverschiebung (PSI) eine lineare Regressionsgleichung gemäß der Formel $\left(N{\left(r\right)}_{\left(mx6\right)}\times {\overline{m}}_{\left(6x6\right)}\right) \times {P%}_{\left(6x1\right)} = mP {\left(t\right)}_{\left(mx1\right)}$
erstellt wird, wobei die Matrix N(t) die Anzahl der Körner jeder Messung innerhalb des Messzeitraumes umfasst, die auf eine der zumindest sechs Gewichtsklassen verteilt werden,
die Diagonalmatrix *m̅* vorgegebene Werte enthält, die das durchschnittliche Korngewicht jeder der zumindest sechs Gewichtsklasse wiedergeben,
der Vektor *mP*(*t*) Werte für die Proteinmasse enthält, die vom Sensor (27) zur Inhaltsstoffanalyse innerhalb eines jeden vorgegebenen Messintervalls für alle im Probenkornstroms (13) enthaltenen Körner (8, 8a) gemeinsam gemessen wurden, und der Vektor P% die zu bestimmende Konzentration an Proteingehalt innerhalb jeweils einer der zumindest sechs Gewichtsklassen umfasst,
erstellt wird, welche für den Vektor P% durch die Auswerteeinheit (21) mittels linearer Regressionsanalyse gelöst wird.

9. Verfahren nach einem der vorangehenden Ansprüche, **dadurch gekennzeichnet, dass** einzelne Körner (8a) aus dem Probenkornstrom (13) mittels einer der optischen Sensoranordnung (23) nachgeordneten Abscheidevorrichtung (25) in Abhängigkeit von der durch die Auswerteeinheit (21) durchgeführten Auswertung von Signalen, die von der optischen Sensoranordnung (23) zur ausgeführten Größenunterscheidung bereitgestellt werden, abgeschieden werden.

10. Verfahren nach Anspruch 9, **dadurch gekennzeichnet, dass** die Körner (8a) in Abhängigkeit vom erntegutabhängig vorgebbaren Schwellwert durch die Abscheidevorrichtung (25) aus dem Probenkornstrom (13) abgeschieden werden.

11. Verfahren nach einem der vorangehenden Ansprüche, **dadurch gekennzeichnet, dass** mittels einer dem optischen Sensor (27) vor- oder nachgeschalteten Sensoreinheit (26), welche einen Sensor oder mehrere Sensoren unterschiedlicher Sensortechnologien umfasst, Parametern bestimmt werden, durch welche auf das Vorhandensein von Bruchkorn, die Korngesundheit, Besatz, Oberflächenbeschaffenheit des Korns (8, 8a) und/oder Farbe des Korns (8, 8a) im Probenkornstrom (13) geschlossen wird.

12. Selbstfahrender Mähdrescher (1) zur Aufnahme und Behandlung von Erntegut (2), der zur Ausführung des Verfahrens nach einem der vorangehenden Ansprüche 1 bis 11 ausgeführt und eingerichtet ist,
- mit einem Kornelevator (10) zur Förderung eines Erntegutstroms (9) von einer Förder- und Reinigungsvorrichtung (4) zu einem Korntank (5),
- mit einer am Kornelevator (10) angeordneten Probenentnahmevorrichtung (12), die zur Entnahme und Bestimmung von Ernteguteigenschaften eines aus dem durch den Kornelevator (10) geführten Erntegutstroms (9) entnommenen Probenkornstromes (13) eingerichtet ist, wobei die in Bypass-Anordnung am Kornelevator (10) angeordnete Probenentnahmevorrichtung (12) eine Messanordnung (20) mit einer Auswerteeinheit (21) aufweist, wobei die Probenentnahmevorrichtung (12) eine rotierend angetriebene Entnahmevorrichtung (14) zur kontinuierlichen Separierung von einzelnen Körnern (8, 8a) als Probenkornstrom (13) aus dem Erntegutstrom (9), eine der Entnahmevorrichtung (14) nachgeordnete optische Sensoranordnung (23) zur Größenerfassung der vereinzelten Körner (8, 8a) im Probenkornstrom (13) und einen der optischen Sensoranordnung (23) nachgeordneten optischen Sensor (27) umfasst, der zur Bestimmung der Inhaltsstoffe des Probenkornstromes (13) ausgeführt und eingerichtet ist.

13. Selbstfahrender Mähdrescher (1) nach Anspruch 12, **dadurch gekennzeichnet, dass** die Entnahmevorrichtung (14) als zumindest ein rotierend angetriebenes Nockenrad (15) mit zumindest einer umlaufenden Nockenreihe (16) oder als ein Zellenrad ausgeführt ist.

14. Selbstfahrender Mähdrescher (1) nach Anspruch 12 oder 13, **dadurch gekennzeichnet, dass** die optische Sensoranordnung (23) zumindest eine Lichtschranke, zumindest einen Reflexionssensor und/oder zumindest einen photoelektrischen Sensor umfasst.

15. Selbstfahrender Mähdrescher (1) nach einem der Ansprüche 12 bis 14, **dadurch gekennzeichnet, dass** die Auswerteeinheit (21) mit einer externen Datenbank (34) verbindbar ist, an die zumindest eine Handlungsempfehlung für einen nachfolgenden Verarbeitungsprozess des Erntegutes (2) übertragbar ist, welche durch die Auswerteeinheit (21) aus Informationen zur Proteinverschiebung (PSI) bestimmt wird, wobei die zumindest eine Handlungsanweisung in der externen Datenbank (34) für autorisierte Dritte abrufbar hinterlegt ist.
